# EUROPEAN PATENT SPECIFICATION

(11) **EP 4 307 338 B1**
(45) Date of publication and mention of the grant of the patent: **27.11.2024**
(21) Application number: 22184545.6
(22) Date of filing: 12.07.2022
(51) Int. Cl.: H01J 37/32, A61B 18/12, H05H 1/46

(54) **RADIO FREQUENCY GENERATOR**
RADIOFREQUENZGENERATOR
GÉNÉRATEUR DE RADIOFRÉQUENCES

(43) Date of publication of application: 17.01.2024
(73) Proprietor: Comet AG, 3175 Flamatt (CH)
(72) Inventor: Galos, Adam, 53937 Schleiden (DE); Grede, André, 3018 Bern (CH); Gruner, Daniel, 79410 Badenweiler (DE); Hartmann, Andreas, 52393 Hürtgenwald (DE); Labanc, Anton, 79238 Ehrenkirchen (DE); Pankratz, Kai, 52457 Aldenhoven (DE); Rüttgers, René, 52152 Simmerath (DE); Sistemich, Roland, 52146 Würselen (DE)
(74) Representative: dompatent von Kreisler Selting Werner - Partnerschaft von Patent- und Rechtsanwälten mbB

(56) References cited:
- WO-A1-2015/023203
- US-A- 5 332 979
- US-A1- 2014 232 483
- US-A1- 2019 313 522

## Description

The present invention relates to a Radio Frequency (RF) Generator in particular for plasma application or plasma process tools.

Plasma processing is a very versatile and precise technique to modify the surfaces of materials, in particular in the manufacturing of semiconductor chips. Such processes are employed multiple times during the entire semiconductor wafer process by using plasma process tools which are typically operated with radio frequency (RF) power with frequencies in the range from 0.3 to 300 MHz and at power levels of 500 W and higher.

In such processing systems, RF power is used to excite gaseous compounds such that free electrons, ions and radicals are formed. Depending on a variety of process parameters, the plasma composition, i.e. the amount of ions, electrons, and various radicals can be controlled precisely. Such plasma processes are used preferably for the deposition of layers onto a semiconductor wafer or for etching the surface of a semiconductor wafer, both with high uniformity and very precisely over the entire surface.

RF power generators are typically set up in 19-inch (482.6-mm) racks together with other electronic equipment. For high throughput, state-of-the-art plasma process tools may comprise several plasma process chambers for parallel processing of several wafers. In addition, often, a plasma process may be operated with two or more RF generators, e. g. one for powering the plasma source and a different one for applying an RF bias to the wafer. Moreover, special plasma processes may be operated simultaneously with several RF frequencies from different RF power generators. In consequence, several RF generators may need to be fitted into one 19-inch (482.6-mm) rack. Since wafer fab space is costly, the size of an RF generator should be as small as possible. For lower RF power levels, e. g. 1-2 kW, two RF generators are typically required to fit into a 19-inch rack side by side, whereas for higher power levels, e. g. more than 5 kW, one generator may occupy the full width of an entire 19-inch (482.6-mm) slot.

Nevertheless, there is a requirement for RF generators, even with power levels higher than 2 kW to fit into an enclosure compatible with 19/2-inch (241.3-mm) size and of 3-5 rack units (U) height (1U = 1.75 inch), that is a height of 133-222 mm, and a length of 450 - 650 mm.

These dimensional restrictions lead to high power density in modern RF generators, and make it necessary that sub-modules, e. g. RF power stages or combiners, are implemented as multilayer or stacked arrangements. In addition, advanced cooling concepts have to be used to remove the heat generated by unavoidable losses, typically in the range of 20% and higher, when RF power is generated.

The term heat sink refers to a heat exchanger that transfers heat from an electronic circuit to a fluid medium, e. g. air or a liquid coolant. One part of the heat sink typically maximizes the contact, and thus heat transfer, to the fluid medium by a cooling structure with enlarged surface area, e. g. by means of cooling fins or spikes or high surface corrugation, in general. The term cold plate refers to a heat exchanger through which a liquid coolant is circulated, typically a thick plate with embedded pipes through which the coolant is flowing. Heat sinks and cold plates are typically made from metallic materials, like aluminum, copper, or metallic alloys, but also ceramics and other materials with good thermal conductivity are possible.

In the following, the term "cooling element" is used for all types of air-cooled heat exchangers with fins or similar enlarged surface area, for liquid-cooled cold plates, and for combinations of a heat spreader with an air-cooled heat sink or liquid-cooled cold plate, including possible enhancements like thermoelectric cooling or heat pipes.

In a high-power RF generator, the individual parts of the RF section, e. g. power amplifying stages and combiners, are typically arranged on a surface of a cooling element as described above, such that the generated heat is efficiently removed and the operating temperature of electronic circuits and components is kept within the safe operating area. In the case of an air-cooled heatsink with fins or spikes, the electronic circuits and components are typically arranged on a surface opposite to the surface with the cooling structure, e. g. on the backside surface of the heatsink with respect to the cooling structure.

To generate high-power RF signals, the signals of several power amplifiers can be combined using various architectures, e. g. in the case of four power stages, two pairs may be combined in a pair of first combiners, and the outputs of the two first combiners are then combined into one signal in a second combining network. In this way, a total output of 5 to 6 kW may be generated by combining 4 power stages with 1.25 - 1.5 kW each via two combining stages. The mechanical arrangement of several power amplifiers and combining networks would typically be done such that the power amplifiers are directly connected to the inputs of the first combiner stages, and the outputs of the first combiner stages are directly connected to the inputs of the second combiner stage. This means that the two combiner stages are arranged in parallel next to each other and their outputs connected directly to appropriately spaced input connectors of a power combiner.

Unfortunately, the described arrangement which could well be realized on the full width of 19 inches (482.6 mm), requires too much space to fit into a 19/2 inches (241.3 mm) wide enclosure. The realization of a small RF power stage with power level above 1kW is difficult especially at frequencies below 50MHz because the size of circuit structures and components scales inversely with frequency. For example, the size of the above mentioned 5-6 kW arrangement would only allow to arrange two 1.25- or kW amplifiers with the first combiner stage in the preferred combination, but the second power combining stage for combining two of the resulting 2.5-kW or 3-kW combinations does not fit into the (241.3-mm) width of the enclosure.

Therefore, the second power combining stage has to be placed elsewhere on the cooling element, for which typically, at power levels > 1 kW, a water-cooled cold plate may be used. This requires longer connections, e. g. 10 cm or more to bridge the distance between submodules. In consequence, there is a need for a connection which fulfills the following requirements:
- Small footprint to fit into the remaining space of the enclosure with its restricted dimensions
- Easy connectivity, e. g. using standard connectors and soldered or screwed connections
- Capability of carrying power > 1 kW
- Low losses and appropriate cooling

A standard solution for connecting RF power circuits over a distance is the use of RF coaxial cables. However, for power levels of several kilowatts, larger cable diameters have to be used to keep the cable temperature within reasonable limits. Such cables require a relatively large space due to their minimum allowed bending radius and the need for RF connectors for best coupling. The use of cables with RF connectors increases cost and requires space for mating connectors on the submodules. In addition, since the cables can get hot in power operation, an efficient cooling, e. g. by a fan, is required.

Another state-of-the-art approach is the use of metal straps, brackets or bridges, e. g. made from copper with silver finish or similar. Such parts can be produced and assembled in a cost-efficient and space-reduced way, for example by using the space above the various PCBs. However, such parts are only sufficient for smaller gaps, e. g. between sub-modules that are located directly side by side. In the case of larger distances between sub-modules which both have 50 Ohm input and output impedance, the connection would have to be impedance matched. This requires that the strap or bridge is running in a certain distance in parallel to a ground plane or in parallel to a second strap of the similar dimension thus forming a structure similar to a transmission line. In consequence, more space and a geometrically more complex arrangement of the connection with particular boundary conditions is required eventually. Moreover, appropriate insulation of the connector against its surroundings has to be ensured to avoid corona and arcing effects towards components in close distance.

Prior art is known from:
US 2019/0313522 A1 describes a circuit support and cooling structure having a three-dimensional, thermally conductive support structure having a plurality of surfaces and a circuit having a plurality of heat generating electrical components disposed on a first portion of the surfaces and interconnected by microwave transmission lines disposed on a second portion of the plurality of surfaces of the thermally conductive support structure.
US 5 332 979 A describes a compact RF power-generator.
US 2014/232483 A1 describes a combiner for an RF power amplifier.
WO 2015/023203 A1 describes an assembly for an RF power coupler in a rack, comprising at least one box shaped power combiner and/or divider, with connectors for in- and output of RF-power, with conductors inside the box, electrically connecting the connectors with at least one center conductor, and with at least one outside conductor. The at least one outside conductor is a box shaped casing.

Thus, it is an objective of the present invention to provide an RF generator which is compact and provides high power output.

The given technical problem is solved by a Radio Frequency (RF) generator according to claim 1.

The RF generator in particular for plasma applications according to the present invention comprises a cooling element. The cooling element may be cooled by active means, e. g. water or air flow, in order to dissipate heat generated by the RF generator. Further, the RF generator comprises at least a first RF power stage having a first output and a second RF power stage having a second output. Therein, the first RF power stage and the second RF power stage are mounted onto a surface of the cooling element. In order to increase the output power of the RF generator, additional RF power stages may be employed and implemented on the same cooling element. Each RF power stage comprises one or more power amplifiers to amplify an RF signal controlled by a driver. Further, by an RF combining network the output of the first RF power stage and the output of the second RF power stage and in particular each further RF power stage are combined to a common RF output. The output of the RF combining network might be provided via an output sensor, e. g. a directional coupler, to the RF output of the RF generator. Therein, the RF combining network is mounted on the same surface of the cooling element as the RF power stages.

According to the present invention, the RF generator comprises an RF distribution element wherein the RF distribution element comprises a printed circuit board (PCB) having a plurality of conductors. The conductors of the RF distribution element are arranged such that the first output of the first power stage is connected to the first input of the RF combining network and the second output of the second power stage is connected to the second input of the RF combining network. Thus, by the conductors of the RF distribution element, the RF signal of the first RF power stage and the RF output signal of the second power stage are provided to the RF combining network to be combined. Therein, the PCB of the RF distribution element is arranged perpendicularly to the surface of the cooling element on which the RF power stages and the RF combining network are mounted. In other words, the side of the RF distribution element with shortest length, i. e. the combined thickness of the PCB and the heat spreader, is in thermal contact with the surface of the cooling element. Thereby a compact arrangement of the RF power stages and the RF distribution element can be achieved. In particular, the RF distribution element itself is compact and requires only little space in the RF generator.

Preferably, the RF generator provides output powers of more than 2 kW and more preferably more than 5 kW.

Preferably, the cooling element of the RF generator is a water-cooled cold plate. In particular for high power RF generators providing output power above 5 kW, water-cooling is mandatory. If it is intended to reduce the size of such high-power RF generators having water-cooling, heat management becomes more challenging due to the dense packing and small distances of the individual components which produce heat.

Preferably, each RF power stage provides more than 1 kW.

Preferably, each RF power stage provides an RF signal with the frequency between 0.3 MHz and 300 MHz.

Preferably, the lengths of each of the provided plurality of conductors is 10 cm or more. Therein the lengths of the conductors are equal to the distance between the outputs of the respective RF power stages and the inputs of the RF combining network.

Preferably, the conductors may have a different length. Alternatively, all conductors have the same lengths.

Preferably, the first RF power stage and the second RF power stage provide in-phase RF signals at their outputs. If the conductors have the same lengths connecting the first RF power stage and the second RF power stage with the RF combining network, due to the same lengths an in-phase signal arrives at the RF combining network for ease of combining these RF signals.

Preferably, the RF generator comprises a housing wherein the RF generator is arranged within this housing. Therein the housing has preferably a standardized 19/2-inch (241.3-mm) format or a 19-inch (482.6-mm) format. Thus, the RF generator is able to fit in a 19/2-inch rack or a 19-inch rack.

Preferably, the RF distribution element is arranged in parallel to a long side of the housing. Therein, preferably the RF distribution element is directly next to the long side of the housing, i. e. no further elements are arranged between the RF distribution element and the housing thereby providing maximum space for the other components of the RF generator such as the RF power stages.

Preferably, the conductors are built as microstrips or striplines. Microstrips are a type of electrical transmission lines which can be fabricated with different technologies and comprise a conductor that is separated from a metallic ground plane by a dielectric layer. A stripline uses a flat strip of metal as conductor that is sandwiched between two metallic planes usually ground planes. The strip and the metallic planes are separated by an insulating or dielectric material.

Preferably, the conductors are formed on a surface of the PCB and/or internally in the PCB. In particular, the internal conductor may be realized by an internal metallic layer.

Preferably, the PCB of the RF distribution element comprises a metallic layer on one surface of the PCB. In particular, if the conductors are built as microstrips, the metallic layer is used as ground. Alternatively, the PCB of the RF distribution element comprises metallic layers on both surfaces of the PCB. In this case the conductors are internal conductors or built as striplines.

Preferably, the at least one metallic layer of the PCB of the RF distribution element is connected to ground via the cold plate of the RF generator.

Preferably, the width of each of the conductors is smaller or equal to the distance between the respective conductor and the metallic layer. Thereby, interference from other modules of the RF generator or the other RF signals in the RF distribution element can be minimized and the influence of metal parts in the vicinity of the RF distribution element on the impedance of the RF conductors can be kept low.

Preferably, the RF distribution element comprises a heat spreader connected to the PCB wherein the heat spreader of the RF distribution element is in contact with the cooling element, typically a cold plate, to transfer heat from the conductors to the cooling element. Therein, by the contact between the heat spreader of the RF distribution element and the cold plate also the metallic layer of the PCB can be connected to ground.

Preferably, the heat spreader is built from metal such as copper or aluminum or an alloy.

Preferably, the heat spreader is connected to the PCB of the RF distribution element by one or more of an adhesive, clamping screws, electrically conductive thermal interface material, solder bond or bonding sheet.

Preferably, the RF distribution element is connected to the cooling element by one or more of an adhesive, clamping screws, electrically conductive thermal interface material, solder bond or bonding sheet. Thereby sufficient heat transfer is enabled from the RF distribution element to the cooling element.

Preferably, the RF distribution element is connected to the first output and the second output of the power stages and the first input and the second input of the RF combining network by brackets and / or screws. The bracket may be soldered to a pad on the RF distribution element, on the combiner, or on the power stage whereby the connection to the corresponding port on the other circuit is made by means of screws. Thereby, easy assembly is facilitated, and a reliable connection is provided between the RF distribution element and the RF power stages at one end and the RF combining network at the other end of the RF distribution element.

The use of solder pads for the attachment of brackets is particularly well compatible with transmission lines designed as microstrips since the solder pads can easily be arranged in the top layer of the PCB where also the microstrips are located. In the case of striplines, contact pads to the signal line are more complex to be realized. Since the signal line is located in an internal layer of the PCB between a top and a bottom metallic layer, contact pads to the signal line have to be formed within openings of the top metallic layer and have to be connected to the signal line layer by means of via holes or arrays of vias.

Preferably, the conductors comprise an even mode impedance and an odd mode impedance of about 50 Ohm with a tolerance of +/-10%. In that way a proper RF energy transfer from the RF power stages to the RF combining network can be achieved and coupling between the conductors minimized.

In the following, the present invention is described in more detail with reference to the accompanying figures.

The figures show:
- Figure 1: a schematic drawing of an RF generator layout according to the present invention,
- Figure 2: a detailed drawing of an RF distribution element according to the present invention, and
- Figure 3: a sectional view of the RF generator of Figure 1 in direction III.

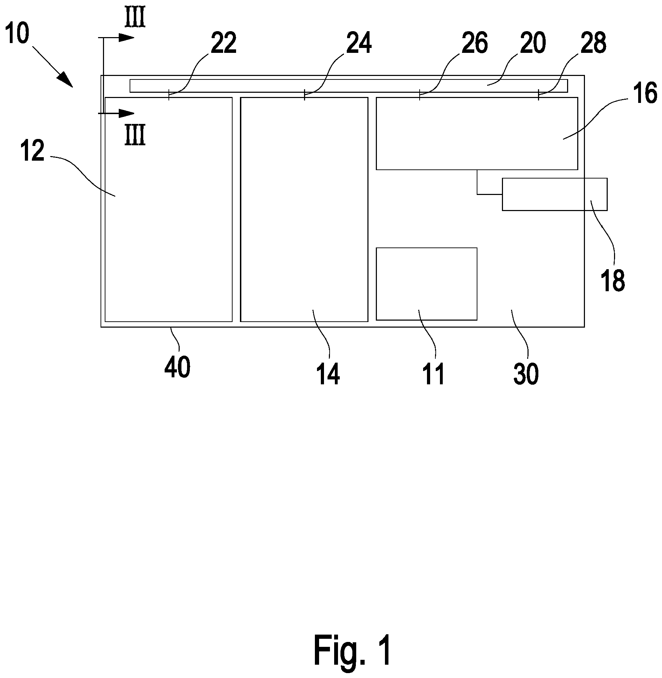

As shown in figure 1, The Radio Frequency (RF) generator 10 according to the present invention comprises a cooling element 30 which is actively cooled. In particular for RF generators generating output powers above 2 kW, usually the cooling element 30 may be a water-cooled cold plate. In particular, the RF generator does not comprise an air-cooling such as cooling fins, a fan, or the like. On a surface of the cooling element 30, a driver 11, a first RF power stage 12 and a second RF power stage 14 are arranged. The power stages 12,14 comprise a power amplifier providing at their outputs an RF signal of more than 1 kW with a frequency between 0.3 MHz and 300 MHz dependent on the specific application. Further, the RF generator 10 comprises an RF combining network 16, wherein the RF signal of the RF power stages 12,14 is combined into a common signal which is then fed to an RF output 18 of the RF generator 10. Also, the RF combining network 16 is arranged on the same surface of the cooling element 30 which in this case is actively cooled.

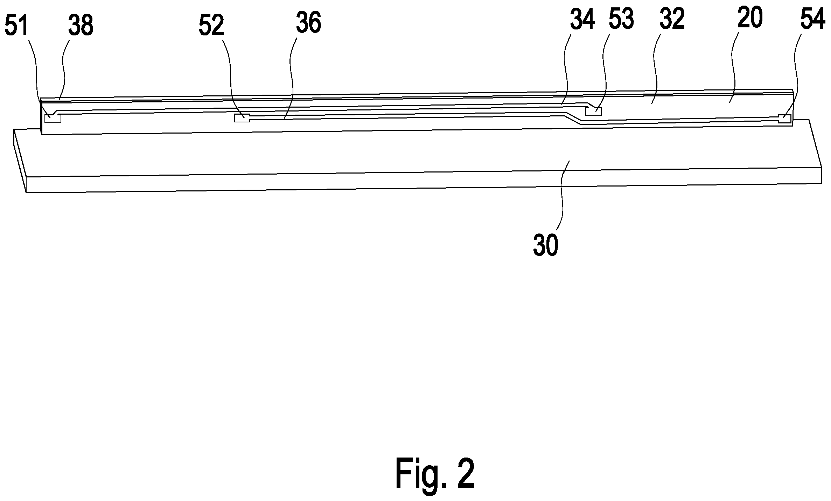

It is desirable to reduce the footprint or building size of the RF generator 10. However, due to the intended frequencies, in particular in the range below 30 MHz, specific frequency dependent electronic component cannot be further reduced. Thus, the spatial requirements increase with lower frequencies. At the same time, with higher powers, more heat is generated which must be effectively dissipated, wherein at the same time reduced space is available on the cooling element. Thus, according to the present invention, the first RF power stage 12 comprises a first output 22 and the second RF power stage 14 comprises a second output 24. The first output 22 and the second output 24 of the RF power stages 12,14 are connected to a first input 26 of the RF combining network 16 and a second input 28 of the RF combining network 16 by an RF distribution element 20. Therein, the RF distribution element 20 is arranged upright or substantially perpendicular to the surface of the cooling element on which the RF power stages 12, 14 and/or the RF combining network 16 are mounted. The RF distribution element 20 as shown in figure 2 comprises a printed circuit board (PCB) 32. The PCB 32 comprises a first conductor 34 arranged to connect the first output 22 of the first RF power stage 12 with the first input 26 of the RF combining network 16. Further, the PCB 32 comprises a second conductor 36 connecting the second output 24 of the second RF power stage 14 with the second input 28 of the RF combining network 16. Therein, further conductors might be implemented into the RF distribution element 20 whereas the number of conductors may correspond to the number of RF power stages. In particular, the conductors 34,36 are built as microstrips or striplines. Due to the arrangement of the RF power stages 12,14 and the RF combining network 16, the lengths of the conductors 34,36 are equal to the distance between the respective outputs 22, 24 of the RF power stages 12,14 and the inputs 26,28 of the RF combining network 16. Preferably, the lengths of the conductors 34,36 of the RF distribution element are 10 cm or longer. As shown in figure 1, the RF distribution element 20 extends along a long side of the housing 40 of the RF generator providing sufficient space for the RF power stages 12,14.

As shown in figure 2, the conductors 34 and 36 comprise contact pads 51, 52, 53, 54 at their ends to which connecting elements, e. g. brackets, can be attached, e. g. soldered. These connecting elements are used to connect the conductors 34, 36 to the outputs 22, 24 of power stages 12, 14, and to the inputs 26, 28 of combining network 18.

Preferably the housing 40 is built as standardized housing mountable into an electronics rack of 19 inches (482.6 mm) width. More preferably, the housing 40 has a width of 19/2 inches (241.3 mm) such that two RF generators 10 can be mounted side by side into an electronics rack of 19 inches (482.6 mm) width.

Preferably, the conductors 34, 36 are of equal length such that signals from the power stages which have a certain phase difference arrive with the same phase difference at the input of the combining network. In particular, signals with identical phase at the power stages can then be combined in phase by the combining network, e. g. a Wilkinson combiner.

If a combining network is used which requires a different value of phase difference between the signals to be combined, e. g. 90° for a 3-dB hybrid combiner, the required phase difference which is present at the outputs of the power stages, is maintained by the RF distribution element 20. In consequence, since the phase difference between the signals is not changed by the RF distribution element 20, an arbitrary distance between RF power stage 12, 14 and RF combining network 16 can be bridged via an RF distribution element 20 wherein the lengths of the individual transmission lines are equal to each other.

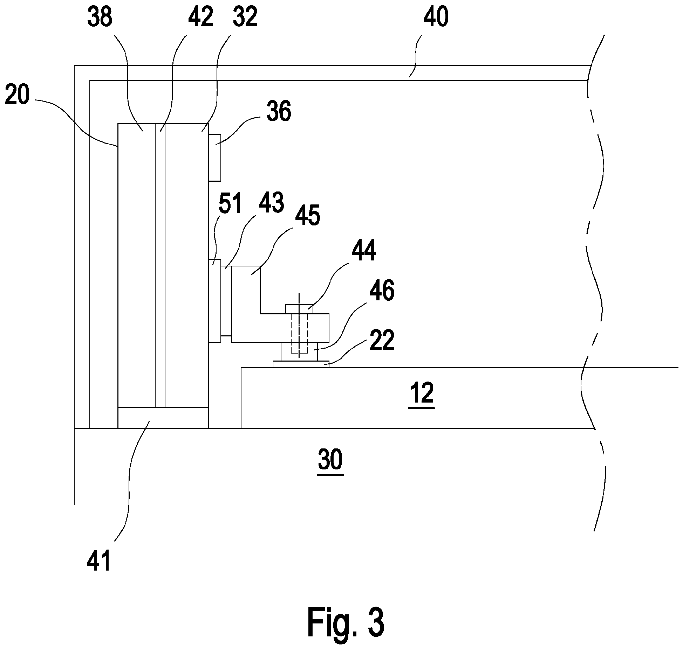

As shown in figures 2 and 3, the RF distribution element 20 and in particular the PCB 32 of the RF distribution element 20 is perpendicular to the cooling element 30, i.e. the surface of the cooling element on which the RF power stages 12,14 and/or the RF combining network 16 are mounted.

The RF distribution element 20 may be attached to the cooling element 30 by a contact element 43 as exemplified in figure 3 and for example configured as adhesive, clamping screws, electrically conductive thermal interface material, solder bond or bonding sheet. In the example of figure 3, the PCB 20 comprises conductors 34,36 on a surface wherein the opposite surface of PCB 32 comprises a metallic layer 42. Therein, the metallic layer 42 is oriented towards the housing 40 of the RF generator. A heat spreader 38 is attached to the metallic layer 42. The heat spreader 38 is connected to the cooling element 30 via the contact element 41. Thereby, the metallic layer 42 is connected to ground, i. e. the cooling element, either indirectly via the heat spreader 38 and the contact element 41 or directly via the contact element 41. Alternatively, the PCB may be built as metallic cladded PCB substrate, e. g. copper cladded PCB substrate. By the heat spreader 38 heat generated in the conductor 34, 36 is efficiently transferred to the cooling element 30 and dissipated.

In the example of figure 3, the conductors 34,36 are built as microstrips. The conductors 34,36 are connected to the respective RF power stages 12,14 via screws and / or brackets connecting the conductors 34,36 to the respective terminals 46 or the RF power stages 12,14. Figure 3 shows the example of contact pad 51 which is connected to conductor 34 and to which a 90°-angled bracket 45 is attached by means of contact element 43, e. g. a solder material. The other end of the bracket 45 is connected via a screw 44 to a terminal 46, e. g. a metal part with an internal thread. Terminal 46 is attached to the output 22 of power stage 12, e. g. via soldering.

As in figure 2, the thermal connection and the electrical ground connection of the RF distribution element 20 is made via contact element 41 which may be a conductive thermal interface material, solder bond or bonding sheet. Alternatively or additionally, screws passing through the top end of the heat spreader 38 and through the contact element 41 into the cooling element 30 may be used to firmly attach the RF distribution element 20 to the cooling element in several locations along the long side of the RF generator.

The arrangement shown in figure 3 has the advantage that it is easy to assemble and disassemble with screws, and all parts can be mounted with high precision such that performance variation from unit to unit is minimized.

By the present invention a compact arrangement of the sub-modules of the RF generator can be achieved wherein the RF distribution element provides an increased flexibility for this arrangement and provides a compact and efficient connection between the sub-modules in order to transfer the RF signals from the respective RF power stages to the RF combining network in a reliable manner.

## Claims

1. A Radio Frequency, RF, generator (10) in particular for a plasma application, comprising:
a cooling element (30);
at least a first RF power stage (12) having a first output (22) and a second RF power stage (14) having a second output (24), both being mounted onto a surface of the cooling element (30);
an RF combining network (16) mounted onto the surface of the cooling element (30) comprising at least a first input (26) and a second input (28);
an RF distribution element (20), wherein the RF distribution element (20) comprises a printed circuit board (32), PCB, having a plurality of conductors (34, 36), wherein the conductors of the RF distribution element are arranged such that the first output (22) of the first RF power stage (12) is connected to the first input (26) of the RF combining network and the second output (24) of the second RF power stage (14) with the second input (28) of the RF combining network (16), respectively,
wherein the PCB (32) of the RF distribution element (20) is arranged perpendicularly to the surface of the cooling element (30).

2. RF generator according to claim 1, wherein the length of each of the plurality of conductors (34, 36) is 10 cm or more.

3. RF generator according to claims 1 or 2, wherein the conductors (34, 36) have the same length.

4. RF generator according to any of claims 1 to 3, wherein the first RF power stage (12) and the second RF power stage (14) provide in-phase RF signals at their outputs (22, 24).

5. RF generator according to any of claims 1 to 4 arrangeable inside a housing, wherein the housing (40) has preferably a standardized 19/2-inch (241.3-mm) format or a 19-inch (482.6-mm) format.

6. RF generator according to claim 5, wherein the RF distribution element (20) is arrangeable in parallel to a long side of the housing (40) and preferably directly next to the long side of the housing (40).

7. RF generator according to any of claims 1 to 6, wherein the conductors (34, 36) are built as microstrips or striplines.

8. RF generator according to any of claims 1 to 7, wherein the conductors (34, 36) are formed on a surface of the PCB (32) and/or internally.

9. RF generator according to any of claims 1 to 8, wherein the PCB (32) of the RF distribution element (20) comprises a metallic layer (42) on one surface of the PCB (32) or on both surfaces of the PCB (32).

10. RF generator according to claim 9, wherein the width of the conductors (34, 36) is smaller or equal to the distance between the conductors (34, 36) and the metallic layer (42).

11. RF generator according to any of claims 1 to 10, wherein the RF distribution element (20) comprises a heat spreader (38) connected to the PCB (32), wherein the heat spreader (38) of the RF distribution element (20) is in contact with the cooling element (30) to transfer heat from the conductors (34, 36) to the cooling element (30).

12. RF generator according to any of claims 1 to 11, wherein the RF distribution element (20) is connected to the cooling element (30) by one or more of an adhesive, clamping screws, conductive thermal interface material, solder bond or bonding sheet.

13. RF generator according to any of claims 1 to 12, wherein the RF distribution element (20) is connected to the first output (22), the second output (24) of the power stages (12, 14), the first input (26) and the second input (28) of the RF combining network (16) by brackets or screws.

14. RF generator according to any of claims 1 to 13, wherein an even mode impedance and an odd mode impedance of the conductors (34, 36) of 50 Ohm +/-10%.

## Patentansprüche

1. Radiofrequenz-, RF, Generator (10), insbesondere für eine Plasmaanwendung, der aufweist:
ein Kühlelement (30);
mindestens eine erste RF-Leistungsstufe (12) mit einem ersten Ausgang (22) und eine zweite RF-Leistungsstufe (14) mit einem zweiten Ausgang (24), die beide auf einer Oberfläche des Kühlelements (30) montiert sind;
ein RF-Kombinationsnetzwerk (16), das auf der Oberfläche des Kühlelements (30) montiert ist und mindestens einen ersten Eingang (26) und einen zweiten Eingang (28) aufweist;
ein RF-Verteilungselement (20), wobei das RF-Verteilungselement (20) eine Leiterplatte (32), PCB, aufweist mit einer Vielzahl von Leitern (34, 36), wobei die Leiter des RF-Verteilungselements so angeordnet sind, dass der erste Ausgang (22) der ersten RF-Leistungsstufe (12) mit dem ersten Eingang (26) des RF-Kombinationsnetzwerks bzw. der zweite Ausgang (24) der zweiten RF-Leistungsstufe (14) mit dem zweiten Eingang (28) des RF-Kombinationsnetzwerks (16) verbunden ist,
wobei die PCB (32) des RF-Verteilungselements (20) senkrecht zu der Oberfläche des Kühlelements (30) angeordnet ist.

2. RF-Generator nach Anspruch 1, wobei die Länge jedes der Vielzahl von Leitern (34, 36) 10 cm oder mehr beträgt.

3. RF-Generator nach Anspruch 1 oder 2, wobei die Leiter (34, 36) dieselbe Länge haben.

4. RF-Generator nach einem der Ansprüche 1 bis 3, wobei die erste RF-Leistungsstufe (12) und die zweite RF-Leistungsstufe (14) gleichphasige RF-Signale an deren Ausgängen (22, 24) aufweisen.

5. RF-Generator nach einem der Ansprüche 1 bis 4, der in einem Gehäuse angeordnet werden kann, wobei das Gehäuse (40) vorzugsweise ein standardisiertes Format von 19/2 Inch (241,3 mm) oder von 19 Inch (482,6 mm) aufweist.

6. RF-Generator nach Anspruch 5, wobei das RF-Verteilungselement (20) parallel zu einer langen Seite des Gehäuses (40) und vorzugsweise direkt neben der langen Seite des Gehäuses (40) angeordnet werden kann.

7. RF-Generator nach einem der Ansprüche 1 bis 6, wobei die Leiter (34, 36) als Mikrostreifen oder Streifenleitungen gebaut sind.

8. RF-Generator nach einem der Ansprüche 1 bis 7, wobei die Leiter (34, 36) auf einer Oberfläche der PCB (32) und/oder intern ausgebildet sind.

9. RF-Generator nach einem der Ansprüche 1 bis 8, wobei die PCB (32) des RF-Verteilungselements (20) eine Metallschicht (42) auf einer Oberfläche der PCB (32) oder auf beiden Oberfläche der PCB (32) aufweist.

10. RF-Generator nach Anspruch 9, wobei die Breite der Leiter (34, 36) kleiner als der oder gleich dem Abstand zwischen den Leitern (34, 36) und der Metallschicht (42) ist.

11. RF-Generator nach einem der Ansprüche 1 bis 10, wobei das RF-Verteilungselement (20) einen Wärmeverteiler (38) aufweist, der mit der PCB (32) verbunden ist, wobei der Wärmeverteiler (38) des RF-Verteilungselements (20) mit dem Kühlelement (30) in Kontakt steht, um Wärme von den Leitern (34, 36) auf das Kühlelement (30) zu übertragen.

12. RF-Generator nach einem der Ansprüche 1 bis 11, wobei das RF-Verteilungselement (20) durch eines oder mehreres aus einem Klebstoff, Klemmschrauben, leitfähigem Wärmeleitmaterial, Lötmittel, oder Klebefolie mit dem Kühlelement (30) verbunden ist.

13. RF-Generator nach einem der Ansprüche 1 bis 12, wobei das RF-Verteilungselement (20) mit dem ersten Ausgang (22), dem zweiten Ausgang (24) der Leistungsstufen (12, 14), dem ersten Eingang (26) und dem zweiten Eingang (28) des RF-Kombinationsnetzwerks (16) durch Halterungen oder Schrauben verbunden ist.

14. RF-Generator nach einem der Ansprüche 1 bis 13, wobei eine Even-Mode-Impedanz und eine Odd-Mode-Impedanz der Leiter (34, 36) 50 Ohm +/- 10 % beträgt.

## Revendications

1. Générateur de radiofréquence, RF, (10), en particulier pour une application plasma, comprenant :
un élément de refroidissement (30) ;
au moins un premier étage de puissance RF (12) ayant une première sortie (22) et un second étage de puissance RF (14) ayant une seconde sortie (24), les deux étant montés sur une surface de l'élément de refroidissement (30) ;
un réseau de combinaison RF (16) monté sur la surface de l'élément de refroidissement (30) comprenant au moins une première entrée (26) et une seconde entrée (28) ;
un élément de distribution RF (20), dans lequel l'élément de distribution RF (20) comprend une carte de circuit imprimé (32), PCB, ayant une pluralité de conducteurs (34, 36), dans lequel les conducteurs de l'élément de distribution RF sont agencés de telle sorte que la première sortie (22) du premier étage de puissance RF (12) est connectée à la première entrée (26) du réseau de combinaison RF et la seconde sortie (24) du second étage de puissance RF (14) à la seconde entrée (28) du réseau de combinaison RF (16), respectivement,
dans lequel le circuit imprimé (32) de l'élément de distribution RF (20) est agencé perpendiculairement à la surface de l'élément de refroidissement (30).

2. Générateur RF selon la revendication 1, dans lequel la longueur de chacun de la pluralité des conducteurs (34, 36) est de 10 cm ou plus.

3. Générateur RF selon les revendications 1 ou 2, dans lequel les conducteurs (34, 36) ont la même longueur.

4. Générateur RF selon l'une quelconque des revendications 1 à 3, dans lequel le premier étage de puissance RF (12) et le second étage de puissance RF (14) fournissent des signaux RF en phase à leurs sorties (22, 24).

5. Générateur RF selon l'une quelconque des revendications 1 à 4, pouvant être agencé à l'intérieur d'un logement,
dans lequel le logement (40) a de préférence un format normalisé de 19/2 pouces (241,3 mm) ou un format de 19 pouces (482,6 mm).

6. Générateur RF selon la revendication 5, dans lequel l'élément de distribution RF (20) peut être agencé parallèlement à un côté long du logement (40) et de préférence directement tout près du côté long du logement (40).

7. Générateur RF selon l'une quelconque des revendications 1 à 6, dans lequel les conducteurs (34, 36) sont construits sous forme de microbandes ou de lignes à ruban.

8. Générateur RF selon l'une quelconque des revendications 1 à 7, dans lequel les conducteurs (34, 36) sont formés sur une surface de la carte de circuit imprimé (32) et/ou à l'intérieur.

9. Générateur RF selon l'une quelconque des revendications 1 à 8, dans lequel la carte de circuit imprimé (32) de l'élément de distribution RF (20) comprend une couche métallique (42) sur une surface de la carte de circuit imprimé (32) ou sur les deux surfaces de la carte de circuit imprimé (32).

10. Générateur RF selon la revendication 9, dans lequel la largeur des conducteurs (34, 36) est inférieure ou égale à la distance entre les conducteurs (34, 36) et la couche métallique (42).

11. Générateur RF selon l'une quelconque des revendications 1 à 10, dans lequel l'élément de distribution RF (20) comprend un répartiteur de chaleur (38) connecté à la carte de circuit imprimé (32), dans lequel le répartiteur de chaleur (38) de l'élément de distribution RF (20) est en contact avec l'élément de refroidissement (30) pour transférer la chaleur des conducteurs (34, 36) vers l'élément de refroidissement (30).

12. Générateur RF selon l'une quelconque des revendications 1 à 11, dans lequel l'élément de distribution RF (20) est connecté à l'élément de refroidissement (30) par un ou plusieurs éléments parmi un adhésif, des vis de serrage, un matériau d'interface thermique conducteur, une liaison de soudure ou une feuille de liaison.

13. Générateur RF selon l'une quelconque des revendications 1 à 12, dans lequel l'élément de distribution RF (20) est connecté à la première sortie (22), à la seconde sortie (24) des étages de puissance (12, 14), à la première entrée (26) et à la seconde entrée (28) du réseau de combinaison RF (16) par des supports ou des vis.

14. Générateur RF selon l'une quelconque des revendications 1 à 13, dans lequel une impédance en mode pair et une impédance en mode impair des conducteurs (34, 36) de 50 Ohm représentent +/-10 %.
